Europäisches Patentamt

(19) European Patent Office  (11) Publication number: **0 231 925**
Office européen des brevets  **B1**

(12) **EUROPEAN PATENT SPECIFICATION**

(45) Date of publication of patent specification :
24.01.90

(21) Application number : 87101393.4

(22) Date of filing : 02.02.87

(51) Int. Cl.⁵ : **A 61 K  7/06**

(54) **Hair care compositions.**

(30) Priority : 07.02.86 US 827904

(43) Date of publication of application :
12.08.87 Bulletin 87/33

(45) Publication of the grant of the patent :
24.01.90 Bulletin 90/04

(84) Designated contracting states :
AT BE CH DE FR GB IT LI LU NL SE

(56) References cited :
US--A-- 3 285 818
CHEMICAL ABSTRACTS, vol. 96, 1982, page 362,
abstract no. 205220a, Columbus, Ohio, US; & JP-A-82
07 405 (POLA CHEMICAL INDUSTRIES, INC.) 14-01-
1982

(73) Proprietor : **Richardson-Vicks, Inc.**
**Ten Westport Road**
**Wilton, CT 06897 (US)**

(72) Inventor : **Yeung, David**
**31 Windermere Lane**
**Stamford Connecticut 06897 (US)**
Inventor : **Hawkins, Geoffrey R.**
**9648 Friar Tuck Drive**
**West Chester Ohio 45069 (US)**

(74) Representative : **Vossius & Partner**
**Siebertstrasse 4 P.O. Box 86 07 67**
**D-8000 München 86 (DE)**

Jouve, 18, rue St-Denis, 75001 Paris, France

**Description**

This invention relates to hair care compositions that are especially useful and effective for increasing hair strength.

The invention provides a cosmetic composition suitable for treating hair, other than a composition for permanently waving hair, comprising a cosmetically acceptable carrier and from about 0.001 to about 5.0 percent by weight, as the total combined amount, of pantethine and pantetheine wherein the ratio between the two is from about 1 : 10 to about 10 : 1.

Hair is made of a complex array of structures, all composed mainly of a protein called keratin. Through its high degree of cross-linking of the disulfide bonds of the amino-acid cysteine, keratin provides hair with resistance to physical deformation. The cuticle of hair, which is extensively cross-linked by cysteine, acts as a protective barrier against the penetration of foreign agents and protects the inner cortex from mechanical or chemical abuses. Thus, this intricate network of disulfide crosslinking is essential to maintain strong and healthy looking hair. The degradation or breakage of these cross-linked disulfide bonds by mechanical and/or chemical treatments, such as hot combing, brushing, permanent waving or sun and weather exposure, may result in the alteration of hair mechanical properties such as a loss in hair tensile strength, thereby rendering the hair weak and easy to break.

It has now been found that the combined use of pantethine and pantetheine in hair care compositions, other than hair perming compositions, strengthens the keratin structure of hair when applied to hair in the customary usage of the particular type of hair care composition, making the so treated hair stronger and more resistant to breakage.

Pantethine is the common name for the known compound, N,N'-[dithiobis(ethyleneiminocarbonyle thylene)]-bis (2,4-dihydroxy-3,3-dimethylbutyramide) ; it is freely soluble in water ; it is synthetized by the formation of a peptide bond between pantothenic acid and cystamine. Panthetheine is the common name for the reduced form of pantethine and is chemically identified as thiobis(ethyleneiminocarbonylethy lene)-bis(2,4-dihydroxy-3,3-dimethylbutyramide). Both are commercially available, for example, from Chugai International Corp., New York, N.Y.

Pantethine itself has been known to be used in certain hair preparations, for example, US-A-3,285,818 (1966) discloses hair tonics containing pantethine ; and Japanese Patent Application Serial No. 55-109849 (1980), publication No. 57-35511 (1982), discloses a shampoo containing pantethine as a skin irritation suppressing agent. The use of pantetheine in hair preparations is not known to applicants. In our copending European Patent Application EP-A-200 208 entitled « Pantethine Component for Hair Permanent Waving », the combined use of pantethine and pantetheine in hair perming compositions is disclosed, which compositions, however, are not embraced within the hair care compositions of the subject invention.

The total combined amount of pantethine and pantetheine used acording to the invention is generally in proportions of from about 0.001 to about 5.0 percent by weight, preferably from about 0.01 to about 1.0 percent by weight and most preferably from about 0.01 to about 0.5 percent by weight, relative to the total weight of the composition, the ratio of pantethine to pantethine being from about 1 : 10 to about 10 : 1 ; preferably from about 1 : 2.5 to about 2.5 : 1 and most preferably about 1 : 1.

The hair care compositions of the invention are suitable in the form of aqueous, alcoholic or aqueous alcoholic solutions or in the form of gels, creams, emulsions, sprays and the like. Any conventional hair cosmetic formulation, other than a hair perming composition, can be used as the cosmetically acceptable carrier of the hair care composition according to the invention. Typical of such cosmetically acceptable carriers for hair are for example, hair shampoos, hair conditioners, hair sprays, hair mousses, hair styling and hair setting gels and compositions, hair tonics, hair creams, hair creme rinses, and the like. These hair cosmetic formulations are well known in the art, containing a variety of cosmetically acceptable ingredients commonly employed in the art, and, as such, need not be discussed individually herein, but particular formulations of various types of hair care compositions will be utilized in the examples hereinafter for purposes of illustration only.

The subject invention thus provides an improved hair care composition, other than a composition for permanently waving hair, wherein the improvement comprises the incorporation into a cosmetically acceptable hair care carrier of the two components, pantethine and pantetheine in the previously defined amounts. Such improved composition provides markedly enhanced hair strengthening properties as compared to the identical composition with either pantethine or pantetheine alone.

In hair care compositions which are meant to be rinsed off with water after appropriate application to the air, such as, for example, aqueous, alcoholic or aqueous alcoholic solutions, gels, emulsions and the like, and, in particular, shampoos, conditioning compositions, rinses and the like, in contrast to those which are meant to be left on the hair after application thereto, for example, hair sprays, mousses, styling gels and the like, it is preferrel that the two components, pantethine and pantetheine, be used in combination with urea. The amount of urea in such compositions may be from about 0.01 to about 5.0 percent by weight and preferably from about 0.01 percent to about 0.5 percent by weight of the total composition.

Hair, upon extension with increasingly applied force, passes through three phases. The first phase is the elastic region which is characterized by reversible extension. The second phase is the yield region, characterized by an irreversible transformation in which the covalent bonds in hair are probably broken. Finally, the third phase corresponds to the breaking point, where complete fiber breakage occurs. It has previously been reported that the breaking point of hair is closely related to hair diameter and may not necessarily be an indication of overall hair damage. Thus, the yield region is the one most likely to correlate with covalent and disulfide bond breakage in hair (i.e., overall hair damage) and, therefore, evaluating the yield slope can provide a measurement of hair damage.

A particularly suitable assessment of overall hair damage utilizes the yield slope analysis technique following the permanent waving of hair as compared to permed hair subsequently treated with the particular test formulation for assessment of the percent reduction in hair damage by such formulation.

The process of perming hair results in chemical damage of hair since disulfide bonds are broken by the reducing operation. The subsequent use of peroxide neutralizer in the conventional two-step perming operation does not reform all the broken bonds and, consequently, the hair to a certain extent remains damaged. Yield slope analysis illustrates the technique used to analyze hair strength correlated to such disulfide bond breakage. Treatment of the thus damaged hair with the composition of this invention results in a marked increase in hair strength or, conversely, reduction in hair damage. Thus, the less damage to the hair occasioned by use of the subject composition, the less change in yield slope will be observed.

With regard to chemically damaged hair such as permed hair, the following testing protocol is utilized. Virgin hair, approximately 2 grams in weight and at least 25 cm long, is bound together at one end. The swatches are then permed with a commercial alkaline wave for 20 minutes. The particular commercial perm product employed in the following examples is Pantene® Salon Wave, Pink Formula (for normal or resistant hair), available from The Pantene Company, a division of Richardson-Vicks Inc., Wilton, CT ; the trademark « Pantene » being owned by said Richardson-Vicks Inc. After thorough rinsing with water to remove excess perm solution, the hair bundles are neutralized with the particular peroxide neutralizer provided with said comercial perm product for 5 minutes and rinsed again. The permed swatches are air-dried for at least 12 hours at ambient temperature to provide the permed control swatches. These damaged hair swatches are then treated with the particular hair product to be treated to assess its ability to increase hair strength.

For shampoo and conditioner treatments, the aforementioned hair swatches are soaked in a 25 % aqueous solution of the test product for 30 minutes and rinsed with warm tap water 29.4-35 °C (85-95 °F) for 30 seconds. This treatment cycle is repeated three times to constitute a total of 4 treatment cycles.

For hair cremes, gels, mousses and emulsion type products, the hair swatches are treated with the undiluted test product by massaging the product on the hair for 2 minutes. Then the hair swatches are rinsed with warm tap water as mentioned previously for four treatment cycles.

For hair sprays, the hair swatches are uniformly sprayed with the product from a distance of 45.72 cm (18 inches) until the hair is uniformly and thoroughly sprayed (about 15 seconds). No water rinsing of the swatches is performed.

The thus treated swatches are again air-dried for at least 12 hours at ambient temperature. Utilizing a tensile strength tester (Instron Model # 1122, manufactured by the Instron Corp., Canton, MA), a single strand of hair is positioned between the two clamps of the tester at a standardized distance of 23 cm. With the instrument set at a crosshead speed of 100 mm/minute and a chart speed of 200 mm/minute, at least 20 single hair strands from each group are extended with a 20 g load setting. The slope of the yield region which correlates with covalent and disulfide bond breakage (i.e., overall hair damage) is measured for each hair. For each group of virgin hair, the mean yield slope for the 20 single hair strand measurements is calculated. The yield slope of untreated virgin hair, which represents totally undamaged hair, and the yield slope of permed hair, which represents chemically damaged hair, provides the « Untreated Normal Hair » and « Permed Hair » measurements, respectively. The mean yield slope of permed hair treated with the composition of this invention is then measured to provide the « Treated Permed Hair » measurements and the percent increase in hair strength is determined according to the formula :

$$\begin{matrix} \% \text{ Increase} \\ \text{in Hair} \\ \text{Strength} \end{matrix} = \frac{\text{Treated Permed Hair} - \text{Permed Hair}}{\text{Untreated Normal Hair} - \text{Permed Hair}} \times 100$$

With regard to hair damaged by sun or ultraviolet radiations, the following testing protocol is utilized. Virgin hair, approximately 2 gms and at least 30 cm long is spread apart in a single layer and taped together at one end. The hair swatch is then exposed to a solar simulator (Kratos Solar Simulator, Model # LH153, Kratos Inc., Westwood, N.J.), adjusted to 20 volts and 30 amperes, at a fixed distance of 64 cm for 3 hours. This solar simulator provides a highly intensified spectrum of light which closely resembles solar radiation and, thus is capable of accelerating the damage to hair produced by sun exposure. After 3 hours, the mean yield slope for at least 20 single hair strands is measured (« Solar Exposed Hair »). In addition, the mean yield slope of at least 20 single hair strands of the unexposed virgin hair is also determined. Thus, the total hair damage induced by solar radiation can be determined as follows :

$$\frac{\text{Total}}{\text{Solar Damage}} = \frac{\text{Yield Slope (Unexposed Hair)}}{- \text{Yield Slope (Exposed Hair)}}$$

To determine the hair strengthening effect of a particular product on solar damaged hair, the following protocol is employed. Virgin hair is first damaged by solar or ultraviolet radiations and the yield slope of unexposed and exposed hair is respectively determined as previously described. The solar damaged hair is then treated with the product to be tested and the mean yield slope of the treated solar damaged hair is determined as before. The percent increase in hair strength is then determined according to the formula:

$$\frac{\text{\% Increase}}{\text{in Hair}} = \frac{\text{Yield}}{\text{Slope}} \cdot \frac{\begin{array}{cc}\text{Treated} & \text{Untreated}\\ \text{Solar} & - \quad \text{Solar}\\ \text{Exposed Hair} & \text{Exposed Hair}\end{array}}{\begin{array}{cc}\text{Untreated} & \text{Untreated}\\ \text{Solar} & - \quad \text{Solar}\\ \text{Normal Hair} & \text{Exposed Hair}\end{array}} \times 100$$

To determine the sun protective effect afforded by various hair products, hair is treated with the particular product to be tested and exposed to the solar simulator as previously described. After 3 hours solar exposure, the mean yield slope is determined and the amount of sun protection resultant from the hair treatment is determined according to the formula:

$$\frac{\text{\% Sun}}{\text{Protection}} = \frac{\text{Yield}}{\text{Slope}} \cdot \frac{\begin{array}{cc}\text{Treated} & \text{Untreated}\\ \text{Solar} & - \quad \text{Solar}\\ \text{Exposed Hair} & \text{Exposed Hair}\end{array}}{\begin{array}{cc}\text{Unexposed} & \text{Untreated}\\ \text{Normal Hair} & - \quad \text{Solar}\\ & \text{Exposed Hair}\end{array}} \times 100$$

The subject compositions may be used to strengthen hair which has been damaged, for example, by virtue of exposure to strong sunlight or ultra-violet light, or due to chemically induced damage such as by the permanent waving of hair, or due to mechanically induced damage such as by brushing, combing, etc. They may also be used to treat normal or undamaged hair as a preventative and strengthener against such damage. Accordingly, the subject invention provides a method of treating hair by application of the herein-described compositions thereto for purposes of repairing hair which has already been damaged and for purposes of protecting hair from such damage.

The following examples are provided to illustrate the subject invention.

Example 1

A shampoo base is prepared by mixing the following ingredients in the amounts indicated:

|  | % w/w |
|---|---|
| Water | 59.5 |
| Sodium lauryl ether sulfate-2 | 40.0 |
| Citric acid | 0.5 |
|  | 100.0 |

Example 2

To the shampoo of Example 1, the indicated amount in percent by weight of pantethine (P) and pantetheine (PE) are added and the percent increase in hair strength after perming and treatment with the respective shampoo is measured according to the previously described treating protocol. For purposes of this example, 0.5 % w/w urea (U) is added to each shampoo formulation other than the shampoo base itself (control) in Tests A, B and C. In Test D, 0.01 %, 0.35 % and 0.5 % by weight urea (U) are utilized.

(See Table page 5)

EP 0 231 925 B1

| | Composition | % Increase in Hair Strength |
|---|---|---|
| Test A: | Shampoo Base (Ex. 1) | 0 (by definition) |
| | + 0.2% P + 0.5% U | 17.9 |
| | + 0.2% PE + 0.5% U | 15.9 |
| | + 0.1% P + 0.1% PE + 0.5% U | 21.6 |
| Test B: | Shampoo Base (Ex. 1) | 0 |
| | + 0.5% P + 0.5% U | 19.6 |
| | + 0.5% PE + 0.5% U | 20.0 |
| | + 0.25% P + 0.25% PE + 0.5% U | 33.4 |
| Test C: | Shampoo Base (Ex. 1) | 0 |
| | + 0.35% P + 0.5% U | 19.2 |
| | + 0.35% PE + 0.5% U | 16.4 |
| | + 0.1% P + 0.25% PE + 0.5% U | 24 |
| | + 0.25% P + 0.1% PE + 0.5% U | 24 |
| Test D: | Shampoo Base (Ex. 1) | 0 |
| | + 0.25% P + 0.25% PE + 0.01% U | 36.5 |
| | + 0.25% P + 0.25% PE + 0.35% U | 33.4 |
| | + 0.1% P + 0.1% PE + 0.5% U | 21.6 |

The foregoing results demonstrate the marked improvement in hair strength resultant from the combined use of pantethine, pantetheine and urea in an aqueous hair shampoo. The results indicate that enhanced activity is observed with the ratio of pantethine to pantetheine being from about 1 : 2.5 to about 2.5 : 1 and the amount of urea being from 0.01 to about 0.5 weight percent.

Exemple 3

In this example, 0,25 % by weight of pantethine (P) and 0.25 % by weight of pantetheine (PE) are added to (A) a commercial mousse, Pantene® Quick Fix Firm Hold Styling Mousse ; (B) a commercial gel, Pantene® Perfect Transition™ Pro-Vitamin Sculpturing Gel ; and (C) a commercial spray, Pantene® Firm Hold Hair Spray. Each of the aforementioned products are available from The Pantene Company, a division of Richardson-Vicks Inc., Wilton, CT, and each of the indicated trademarks are owned by said Richardson-Vicks Inc. The percent increase in hair strength after perming and treatment with the respective compositions is measured according to the previously described protocol.

| Composition | % Increase in Hair Strength |
|---|---|
| A. Mousse | 27.0 |
| B. Gel | 27.0 |
| C. Spray | 27.0 |

5

Example 4

In this example, the percent increase in hair strength is measured against solar-induced damage. To each of the commercial mousse, gel and hair spray products identified in Example 3 is added 0.25 % by weight of pantethine and 0.25 % by weight of pantetheine. Each of the respective products are then treated according to the previously described protocol for assessing hair damage upon exposure to a solar simulator and the following results are obtained :

| Composition | % Increase in Hair Strength |
|---|---|
| A. Mousse | 30.0 |
| B. Gel | 23.0 |
| C. Spray | 28.0 |

Example 5

The following example demonstrates the ability of combined pantethine and pantetheine to protect hair from solar damage with effective concentrations as low as 0.001 % and 0.01 % by weight. The testing procedure is the previously described protocol for hair exposed to a solar stimulator. All treatments are with the shampoo base of Example 1 (control).

| Composition | % Sun Protection |
|---|---|
| Shampoo Base (Ex.1) | 0 (by definition) |
| Test A: + 0.001% P + 0.5% U | 54 |
| + 0.001% PE + 0.5% U | 55 |
| + 0.0005% P + 0.0005% PE + 0.5% U | 88 |
| Test B: + 0.01% P + 0.5% U | 82 |
| + 0.01% PE + 0.5% U | 71 |
| + 0.005% P + 0.005% PE + 0.5% U | 92 |
| Test C: + 0.0055% P + 0.5% U | 57 |
| + 0.0055% PE + 0.5% U | 59 |
| + 0.0005% P + 0.005% PE + 0.5% U (ratio of P:PE = 1:10) | 67 |
| + 0.005% P + 0.0005% PE + 0.5% U (ratio of P:PE = 10:1) | 76 |
| Test C: + 0.0005% P + 0.0005% PE | 46 |
| + 0.0005% P + 0.0005% PE + 0.5% U | 88 |
| + 0.005% P + 0.005% PE | 49 |
| + 0.005% P + 0.005% PE + 0.5% U | 92 |

The foregoing data demonstrate the marked increase in sun protection (i.e., increase in hair strength) obtained with the combined use of pantethine and pantetheine as compared to either component alone, which increase is indicative of a synergistic action of the two components when used together. The data also demonstrate the beneficial use of urea, a hair penetration enhancer, on enhancing the activity of combined pantethine and pantetheine in accordance with this invention.

## Claims

1. A hair care composition comprising a cosmetically acceptable hair care composition other than a composition for permanently waving hair and from about 0.001 to about 5.0 combined weight percent of pantethine and pantetheine in a ratio of from about 1 : 10 to about 10 : 1.

2. The composition of claim 1 wherein said combined weight percent is from about 0.01 to about 1.0.

3. The composition of claim 2 wherein said combined weight percent is from about 0.01 to about 0.5.

4. The composition of any one of claims 1 to 3 wherein said ratio is from about 1 : 2.5 to about 2.5 : 1.

5. The composition of claim 4 wherein said ratio is about 1 : 1.

6. The composition of any one of claims 1 to 5 which is a hair shampoo, a hair mousse, a hair gel or a hair spray.

7. The composition of any one of claims 1 to 6 wherein the composition is capable of being rinsed off the hair with water after application thereto and contains in addition from about 0.01 to about 5.0 weight percent of urea.

8. The composition of claim 7 wherein the percent of said urea is from about 0.01 to about 0.5.

9. The use of the composition of any one of claims 1 to 8 for treating hair.

## Patentansprüche

1. Haarpflegemittel-Zusammensetzung, umfassend eine kosmetisch verträgliche Haarpflege-Zusammensetzung, die keine Zusammensetzung für die Dauerwellung von Haar ist, und von etwa 0,001 bis etwa 5,0 kombinierte Gwichtsprozent Pantethin und Pantethein in einem Verhältnis von etwa 1 : 10 bis etwa 10 : 1.

2. Zusammensetzung nach Anspruch 1, in der die kombinierten Gewichtsprozent etwa 0,01 bis etwa 1,0 betragen.

3. Zusammensetzung nach Anspruch 2, in der die kombinierten Gewichtsprozent etwa 0,01 bis etwa 0,5 betragen.

4. Zusammensetzung nach einem der Ansprüche 1 bis 3, in der das Verhältnis etwa 1 : 2,5 bis etwa 2,5 : 1 beträgt.

5. Zusammensetzung nach Anspruch 4, in der das Verhältnis etwa 1 : 1 beträgt.

6. Zusammensetzung nach einem der Ansprüche 1 bis 5, welche ein Haarshampoo, ein Haarschaum, ein Haargel oder ein Haarspray ist.

7. Zusammensetzung nach einem der Ansprüche 1 bis 6, in der die Zusammensetzung in der Lage ist, aus dem Haar mit Wasser nach ihrer Anwendung ausgespült zu werden, und zusätzlich etwa 0,01 bis etwa 5,0 Gew.-% Harnstoff enthält.

8. Zusammensetzung nach Anspruch 7, wobei der Prozentanteil des Harnstoffs etwa 0,01 bis etwa 0,5 beträgt.

9. Verwendung der Zusammensetzung nach einem der Ansprüche 1 bis 8 zur Haarbehandlung.

## Revendications

1. Une composition pour les soins des cheveux comprenant une composition pour soins capillaires acceptable en cosmétique autre qu'une composition pour l'ondulation permanente des cheveux et de 0,001 environ à 5,0 % en poids combinés de pantéthine et de pantéthéine dans un rapport de 1 : 10 environ à 10 : 1 environ.

2. La composition selon la revendication 1, dans laquelle ledit pourcentage en poids combiné est de 0,01 environ à 1,0 environ.

3. La composition selon la revendication 2, dans laquelle ledit pourcentage en poids combiné est de 0,01 environ à 0,5 environ.

4. La composition selon l'une quelconque des revendications 1 à 3, dans laquelle ledit rapport est de 1 : 2,5 environ à 2,5 : 1 environ.

5. La composition selon la revendication 4, dans laquelle ledit rapport est de 1 : 1 environ.

6. La composition selon l'une quelconque des revendications 1 à 5, constituée par un shampooing pour cheveux, une mousse pour cheveux, un gel pour cheveux ou une pulvérisation pour cheveux.

7. La composition selon l'une quelconque des revendications 1 à 6, dans laquelle la composition est susceptible d'être enlevée par rinçage des cheveux à l'eau après application sur ces derniers et contient en outre de 0,01 environ à 5,0 % environ en poids d'urée.

8. La composition selon la revendication 7, dans laquelle le pourcentage de ladite urée est de 0,01 environ à 0,5 environ.

9. L'utilisation de la composition selon l'une quelconque des revendications 1 à 8 pour le traitement des cheveux.